Europäisches Patentamt

(19) European Patent Office

Office européen des brevets

(11) **EP 0 897 000 A1**

(12) **EUROPEAN PATENT APPLICATION**
published in accordance with Art. 158(3) EPC

(43) Date of publication:
17.02.1999 Bulletin 1999/07

(21) Application number: 97919668.0

(22) Date of filing: 24.04.1997

(51) Int. Cl.$^6$: **C12N 11/08**, C02F 3/00,
C12M 1/40

(86) International application number:
PCT/JP97/01420

(87) International publication number:
WO 97/41216 (06.11.1997 Gazette 1997/47)

(84) Designated Contracting States:
**DE FR GB**

(30) Priority: **01.05.1996 JP 135828/96**
**22.04.1997 JP 120198/97**

(71) Applicant: **KANEBO LTD.**
**Sumida-ku, Tokyo 131 (JP)**

(72) Inventors:
• **INOUE, Jun**
**Sashima-gun, Ibaraki 306-02 (JP)**

• **SASAKI, Yasuoki**
**Sashima-gun, Ibaraki 306-02 (JP)**
• **MURAYAMA, Osamu**
**Sashima-gun, Ibaraki 306-02 (JP)**
• **SEKIGAWA, Tetsuo**
**Setagaya-ku, Tokyo 157 (JP)**

(74) Representative:
**Reinhard - Skuhra - Weise & Partner**
**Postfach 44 01 51**
**80750 München (DE)**

(54) **MICROORGANISM CARRIER AND PROCESS FOR PRODUCTION THEREOF**

(57) A porous microbial support which is excellent in a wear resistance, a weatherability (light resistance), a resistance to microbial degradation, a fluidity and a microbial affinity. A microbial support of a polyvinyl acetal porous material which is a particulate sponge of polyvinyl formal with a size in a hydrous condition of from 1 mm to 20 mm, and which has an apparent specific gravity in a hydrous condition of from 1.0 to 1.2, a porosity of from 50 to 98%, a degree of acetalization of from 30 to 85 mol% and a pore diameter of from 20 to 300 μm. This microbial support is excellent in a wear resistance, a weatherability (light resistance), a resistance to microbial degradation, a fluidity and a microbial affinity, and can appropriately be used in various bioreactors including a fluidized-bed type sewage treatment unit in particular.

EP 0 897 000 A1

## Description

Technical Field

The present invention relates to a microbial support which is used in, for example, a microbial catalytic sewage treatment unit for treating a sewage having a high biochemical oxygen demand (BOD) or chemical oxygen demand (COD), such as a drainage, an industrial waste water or a human waste water, and a bioreactor in which medications, foods and the like are subjected to a biochemical reaction using microbes to obtain reaction products, and to a process for producing the same. More specifically, the present invention relates to a microbial support which can appropriately be used for a fluidized-bed type in particular, and a process for producing the same.

Background Art

A method in which microbes or enzymes are filled in a reaction vessel and a reaction by the microbes or the enzymes is utilized to obtain a product is a so-called bioreactor, and it has been so far industrially applied to the field of producing foods, medications or chemical products or in the field of a drainage, a waste water, an exhaust gas and the like. Further, in recent years, to efficiently conduct the treatment, a variety of methods in which this biomicrobial catalyst is charged within a reaction vessel at a high density have been studied.

The most typical method is one in which microbes are carried on a particulate support, and this method is roughly classified into the following two. One of them is a microbial film method in which a microbial film obtained by carrying microbes on a surface of a support is used. Another is an entrap-immobilization microbial method in which microbes are immobilized within a support. Further, as the material of this support, there are a high-molecular material and an inorganic material. Still further, with respect to the use form of the support, there are a fixed-bed type in which a support is fixed within a reaction tank and a fluidized-bed type in which a support is used while being fluidized.

Since a fluidity and a specific gravity are important to the support used in the fluidized-bed type, not an inorganic support but a high-molecular particulate support is generally used. A variety of methods using as a material of this support a particulate gel such as a polyvinyl alcohol gel (PVA gel), an acrylamide gel or a polyethylene glycol gel, a porous particulate material such as polyethylene, polyurethane, polyvinylidene chloride or cellulose and an inorganic particulate material such as ceramics, active carbon or sand have been proposed.

Further, in the microbial film method, microbes can be carried on the surface of the film. Accordingly, this method is characterized in that the immobilization is simple. However, it involves problems that it takes much time to adhere microbes and grow the same, microbes that are easily adhered to a support are preferentially adhered and microbes adhered to the surface are peeled off therefrom. On the other hand, in the entrap-immobilization microbial method, microbes are incorporated within a support, so that any microbes can be immobilized in any amounts.

As a material of the support used in this entrap-immobilization microbial method, a wide variety of materials can be used. Since organic materials or phosphorus or nitrogen compounds which are materials to be treated in a waste water have to be permeated through a hydrous gel and treated in contact with microbes immobilized within the support, a gel-like support of acrylamide, agar, sodium polyacrylate, polyethylene glycol, carrageenan or a photo-setting resin is mainly used.

The gel-like support such as a PVA gel, an acrylamide gel or a polyethylene glycol gel has an excellent affinity for microbes, but exhibits, because it is a gel-like material, a low mechanical strength, and is especially very poor in a wear resistance. When the support is used in a fluidized-bed type, there are problems that it tends to be worn out owing to rubbing between supports which occurs in the fluidization thereof or owing to rubbing with an inner wall of a reaction tank and the life of the support is short.

On the other hand, the porous particulate material such as polyethylene or polyurethane is not so good in a mechanical strength, a durability and a weatherability, and there is a high likelihood that when the material is used for a long period of time, it collapses. Further, a relatively strong polyether-type polyurethane is expensive. A support in which a natural product such as cellulose or the like is used has an excellent affinity for microbes, but is itself susceptible to decomposition due to microbes because it is a natural material, and the life thereof is short. Still further, an inorganic particulate material is poor in a fluidity, and is not appropriate for a system like a fluidized-bed type in which a support is fluidized while being stirred.

In addition, the material of the support used in the entrap-immobilization microbial method has, as stated earlier, a low strength because it is itself a gel-like material, and a wear resistance is notably poor. For this reason, there was a problem that the life of the support is generally extremely short compared to that of a sponge used in a microbial film method, though it varies depending on use conditions such as an immobilization material, a type of a reaction tank, a filling rate and the like.

Thus, there are a wide variety of supports for microbes. However, there has not yet been any noteworthy proposal of supports including the known supports, that meet all of properties required for a support, such as a microbial affinity, a specific gravity, a wear resistance, a weatherability (light resistance) and a resistance to microbial degradation.

The present invention has been made to eliminate the above-mentioned problems, and is to provide a microbial support which is excellent in a wear resistance, a weatherability (light resistance), a resistance to microbial degradation, a fluidity and a microbial affinity.

Disclosure of the Invention

In order to solve the above-mentioned problems, investigations have been assiduously conducted, and a knowledge has been acquired consequently that when using a porous material composed of a polyvinyl acetal, namely a polyvinyl acetal porous material, especially a polyvinyl formal (PVF) porous material, a microbial support which is excellent in a wear resistance, a weatherability (light resistance), a resistance to microbial degradation, a fluidity and a microbial affinity can be provided. The present invention is a microbial support characterized in that a polyvinyl acetal porous material is used as the microbial support.

Accordingly, when a polyvinyl acetal porous material, especially a polyvinyl formal (PVF) porous material is used as a microbial support, a microbial support which is excellent in a wear resistance, a weatherability (light resistance), a resistance to microbial degradation, a fluidity and a microbial affinity can be formed.

Best Mode For Carrying Out the Invention

It is presumably for the following reasons that the polyvinyl acetal porous material, especially, the polyvinyl formal (PVF) porous material can give the microbial support which is excellent in the wear resistance, the weatherability (light resistance), the resistance to microbial degradation, the fluidity and the microbial affinity.

That is, the main reasons are considered to be that the polyvinyl acetal porous material, especially the polyvinyl formal (PVF) porous material contains a large number of OH-groups and is therefore hydrophilic so that it is excellent in an affinity for microbes, that the resistance to microbial degradation which PVA lacks can be imparted thereto by acetalization or formalization of PVA, that since the material has a resin structure, a wear resistance can be exhibited, and that since the material is porous, the specific gravity can be close to the range of from 1.01 to 1.1 and the fluidity can be exhibited.

The microbial support of the present invention has preferably a structure of a sponge formed of a polyvinyl acetal porous material. Consequently, a microbial support having an excellent fluidity can be provided while maintaining a wear resistance.

It is advisable that the apparent specific gravity in the hydrous condition of the microbial support of the present invention is adjusted to between 1.0 and 1.2.

When the apparent specific gravity in the hydrous condition is less than 1.0, the support only floats even when it is charged into a treatment tank, making the treatment difficult. When it exceeds 1.2, the support is liable to sink. In this point, the support lacks the fluidity. Accordingly, in the microbial support of the present invention in which the apparent specific gravity in the hydrous condition of the polyvinyl acetal porous material is between 1.0 and 1.2, an appropriate fluidity can be exhibited well when it is used in a particulate porous material of a fluidized-bed type in particular.

It is advisable that the specific gravity of the microbial support in the present invention when a liquid to be treated is filled in pores of the porous material is closer to that of the liquid to be treated itself. When calculation is conducted on the assumption that the liquid to be treated is water, the apparent specific gravity in the hydrous condition is 1.0 or more, and it is most appropriate that the apparent specific gravity is as close to 1.0 as possible. However, actually, the most appropriate apparent specific gravity in the hydrous condition as the polyvinyl acetal porous material is, as noted above, between 1.0 and 1.2, preferably between 1.01 and 1.1. The most appropriate true specific gravity of the material itself is in the range of from 1.24 to 1.28, preferably in the range of from 1.25 to 1.26. When the polyvinyl acetal porous material having the specific gravity within these ranges is used as a microbial support, especially as a particulate porous material of a fluidized-bed type, an excellent fluidity is exhibited.

It is advisable that the microbial support of the present invention is a particulate material having a size in a hydrous condition of from 1 mm to 20 mm. As a result, the fluidity is improved, and a microbial treatment ability can be exhibited. Even when it is used in a sewage treatment unit having a recovery filter of a particulate microbial support, there is no fear that microbes might be passed through the recovery filter and flow out of a discharge outlet of a treated waste water in the unit. Thus, microbes can be retained at high concentrations.

When the size of the particulate material exceeds 20 mm, not only is the fluidity decreased, but also the effective surface area in which to support microbes is decreased, so that the microbes are hardly maintained at high concentrations and the microbial treatment ability is decreased. In this respect, the smaller the particle diameter of the particulate material, the better the fluidity. In the fluidized-bed type reactor, the energy for fluidizing the support is diminished to improve the treatment ability. However, when the energy is too small, specifically when the size of the particulate material is less than 1 mm, there is a fear that when using a sewage treatment unit having a recovery filter of a particulate microbial support or the like, microbes might be passed through the recovery filter and flow out of the discharge outlet of the treated waste water in the unit, with the result that microbes are hardly maintained at high concentrations. In this connection, it is considered to more decrease a size of a slit mesh of a recovery filter, for example, a wedge wire screen with a mesh size of 1.5 mm which is disposed in a treated liquid outlet. However, since fine particles, microbes, viscous matters of microbes and the like in a waste water are adhered to the slit and clog the same, the mesh size of the filter is naturally limited, and the optimal size of the particulate polyvinyl acetal porous material is determined in view of the relationship to the recovery filter of the particulate microbial support.

The microbial support of the present invention can be used in both of a fixed-bed type and a fluidized-bed type. In this respect, the form of the product is not particularly limited, examples thereof being a block, a film, a sheet, a ribbon and the like. Especially when it is used in a bioreactor such as a fluidized-bed type sewage treatment unit, a particulate form is preferable as mentioned above. The form can be particulate, and various forms, such as spherical, cubic, rectangular parallelopiped and triangular pyramid forms are available. In the spherical form, it is most appropriate that the particle diameter is between 1 and 20 mm. In the cubic form, it is most appropriate that the maximum size is between 1 and 20 mm.

In the microbial support of the present invention, it is advisable that the polyvinyl acetal porous material has communicating pores of which the porosity is between 50 and 98%. Consequently, a microbial support which is excellent in a wear resistance, a weatherability (light resistance), a resistance to microbial degradation, a fluidity and a microbial affinity can be formed.

That is, the polyvinyl acetal porous material of the present invention can be produced by, for example, adding a pore forming agent to a polyvinyl alcohol aqueous solution and reacting the mixture with an aldehyde in the presence of an acid catalyst. In this case, it has been found that when conditions such as a type, an amount and the like of a pore forming agent are changed, a microbial support which is excellent in a wear resistance, a weatherability (light resistance), a resistance to microbial degradation, a fluidity and a microbial affinity can be formed in the case of communicating pores having a porosity of from 50 to 98%, especially an average porosity of 90%. When the porosity is less than 50%, the apparent specific gravity is increased to decrease a water content. Expecially when the product is used as a particulate porous material in a fluidized-bed type, it is easy to sink but hard to float, decreasing a fluidity in a treatment tank. Meanwhile, when the porosity exceeds 98%, it is undesirous in view of a wear resistance, a weatherability (light resistance) and a resistance to microbial degradation.

The above-mentioned range of the porosity is specified especially on the basis of a porous material made only of a polyvinyl acetal, especially polyvinyl formal. Accordingly, when another material having a specific gravity of, for example, less than 1.0 is mixed or compounded with a polyvinyl acetal, the porosity is not particularly limited to the above-mentioned range. For example, when the starting material is kneaded with a powder, fibers, polyethylene, polypropylene, oil or paraffins having a low specific gravity during the production of a polyvinyl formal porous material, it is possible to adjust the porosity or the specific gravity. Especially, the use of hollow microcapsules can easily decrease the specific gravity of the support.

The degree of acetalization of the polyvinyl acetal porous material is not particularly limited. A polyvinyl acetal porous material having a degree of acetalization of from 30 to 85 mol%, preferably from 45 to 70 mol% is appropriate. Above all, polyvinyl formal having a degree of formalization of from 30 to 85 mol%, preferably from 45 to 70 mol% is appropriate. In the present invention, a microbial support in which a degree of formalization of a polyvinyl formal porous material is from 30 to 85 mol%, most preferably from 45 to 70 mol% is further preferable.

A soft microbial support having a wear resistance can be provided by adjusting a degree of acetalization. When the degree of acetalization is less than 30 mol%, a degree of crosslinking is low to give a poor strength, and a fastness to rubbing is decreased. Accordingly, when the support is used as a particulate porous material in a fluidized-bed type in particular, it tends to be worn out owing to rubbing of supports that occurs during fluidization or owing to rubbing with an inner wall of a reaction tank, decreasing the life of the support. Further, it is undesirous in that a resistance to microbial corrosion is decreased. Still further, the support is hard to handle in the production. Meanwhile, when the degree of acetalization exceeds 85 mol%, the porosity is decreased, and the apparent specific gravity is increased to decrease the water content. Especially when this support is used as a particulate porous material in a fluidized-bed type, it is easy to sink but hard to float, decreasing a fluidity within the treatment tank. Furthermore, since a hydrophilic nature is decreased with the decrease in the residual amount of the hydroxyl group, this is particularly unwanted. Moreover, since an impact resilience in wetting is decreased, the support is also unwanted in view of a water absorption and a durability. Especially when a compressed product is formed by a dry-compression step, this product is hardly or not returned to its original shape even through wetting in a treatment tank. Accordingly, the product is susceptible to a compression set, and it is impossible to provide a compressed product. In this respect, in the case of a polyvinyl acetal porous material having a degree of acetalization of from 30 to 85 mol%, preferably from 45 to 70 mol%, especially a polyvinyl formal porous material having a degree of formalization of from 30 to 85 mol%, preferably from 45 to 70 mol%, it is good in a resistance to microbial corrosion and excellent in a strength, and has a great fastness to rubbing.

Accordingly, when the support is used as a particulate porous material in a fluidized-bed type, the life of the support is especially improved. Further, when it is used as a particulate porous material in a fluidized-bed type, it is possible to retain a preferable porosity and a preferable apparent specific gravity in a hydrous condition by which the support is easy to sink, float and fluidize in a treatment tank. Further, since a hydrophilic nature is good, an excellent fluidity is exhibited. Moreover, an impact resilience in wetting is also increased. Even when a compressed product is provided by dry-compression, the product is less susceptible to a compression set. When this product is wetted in a treatment tank or the like, it is returned to the original shape. Accordingly, a compressed product can be formed, and a transportability of a particulate porous material can rapidly be improved. When the product is wetted in a treatment tank or the like, this is compatible with a liquid to be treated and absorbs water to restore its original shape. Accordingly, it can immediately sink, float and be fluidized in the treatment tank. It is not necessary to wait until hydrous particles are provided, and a treating time can therefore be shortened extremely.

The polyvinyl acetal porous material in the present invention has a large number of pores with such a pore diameter that water or air of from 20 to 300 μm can freely come in and out of pores, and it is different from a mere gel having a network structure at a molecular level. When it is used in a fluidized bed as a microbial support, an organic material, a phosphorus compound or a nitrogen compound in water can freely come in and out of the pores having this diameter and easily pass therethrough. Further, a microbial support having a large number of pores with a pore diameter of from 20 to 300 μm is preferable in that microbes can easily form a film and a microbial film formed on the surface of the support is hardly peeled off.

A sponge formed of the polyvinyl acetal porous material in the present invention has a large number of pores with such a diameter that water or air can likewise freely come in and out of the pores, and it exhibits an appropriate resilience of from 2 to 200 x $10^3$ N/m$^2$ with a 50% compression stress on condition that it is swollen with water in a water content of 50%. This appropriate resilience contributes to exhibiting a good wear resistance when a support is fluidized.

The polyvinyl acetal porous material of the present invention sinks, floats and is fluidized well within a treatment tank as a hydrous porous material through aeration only by being charged into a fluidized-bed type treatment tank. Further, since it has a good affinity for microbes as a material, microbes are adhered thereto, making it possible to conduct excellent microbial treatment. Further, since a wear resistance is good, it is hardly worn out owing to rubbing of supports which occurs in fluidization thereof or owing to rubbing with an inner wall of a reaction tank. Further, a mechanical strength is high, a weatherability (light resistance) and a resistance to microbial degradation are excellent, and a life of a support is prolonged.

The present invention is, as stated above, the microbial support in which microbes are immobilized on the surface of, and/or in the pores of, the polyvinyl acetal porous material.

The above-mentioned microbial support is preferable, as stated above, when microbes are adhered to the surface of the support and also to the pores of the porous material. Even when the support is pulverized, the fluidity is excellent, and it is most appropriate for the fluidized-bed type. However, with respect to the entrap-immobilization microbial method, 1) microbes can be maintained at high concentrations, and a waste water can be treated at high speed, 2) treatment of specific substances or recovery of organic materials can be conducted by immobilizing specific microbes, and 3) an amount of a sludge formed can be reduced. Accordingly, it is advisable that the support can be applied to the above-mentioned microbial film method and also to the entrap-immobilization microbial method. Accordingly, a microbial support has been developed in which microbes are positively immobilized within pores of a porous material using a microbe immobilization agent.

That is, the present invention is a microbial support in which microbes are entrapped and immobilized on the surface of, and/or in the pores of, the polyvinyl acetal porous material with a microbe immobilization agent.

Consequently, a wear resistance, a weatherability (light resistance), a resistance to microbial degradation, a fluidity and a microbial affinity are excellent. Microbes can be maintained at high concentrations, and a waste water be treated at high speed. Treatment of specific substances or recovery of organic materials is enabled by immobilizing specific microbes, and an amount of a sludge formed can be reduced. That is, the microbial support can be provided which makes use of merits of the microbial film method and the entrap-immobilization microbial method and conquers the defects of both the microbial film method and the entrap-immobilization microbial method.

As the microbe immobilization agent here, a variety of agents can be employed, and it is not particularly limited. However, a microbe immobilization agent composed mainly of sodium alginate is preferable. When sodium alginate is used as a main component, microbes are easily filled and immobilized well in the polyvinyl acetal porous material, especially in the polyvinyl formal porous material, and it has a good affinity for microbes. Further, it is especially good in view of a wear resistance.

Microbes can be entrapped and immobilized using the microbe immobilization agent by, for example, dipping the polyvinyl acetal porous material into a mixed solution of the microbe immobilization agent containing the microbes and insolubilizing the above-mentioned microbe immobilization agent within the pores of the above-mentioned porous material.

The microbial support of the present invention can be applied as a support of a fluidized bed or a fixed bed, as stated above in both of the above-mentioned entrap-immobilization type (entrap-immobilization method) and the above-mentioned non-entrap immobilization type (microbial film method), and also to various bioreactors including a sewage treatment unit.

Especially, the microbial support of the present invention can preferably be used in a fluidized-bed type sewage treatment unit in which the treatment is conducted such that the particulate microbial support of the present invention floats, sinks and is convected within a treatment tank.

A fluidized-bed type bioreactor can be applied if the above-mentioned support is brought into contact with the liquid to be treated to conduct biological treatment or chemical treatment. Specifically, it can be applied to a device which conducts a chemical reaction such as, other than decomposition of organic materials, oxidation and reduction, for example, nitrifying or denitrifying, addition, substitution, conversion or elimination.

The microbial support of the present invention takes the form of a block, a film, a sheet, a ribbon or the like in both of the above-mentioned entrap-immobilization type (entrap-immobilization method) and the above-mentioned non-entrap immobilization type (microbial film method) as noted above, and its form is not limited. In order that the support is easily used by being filled in a sewage treatment unit or a bioreactor and it is conveniently used in a fluidized bed, the particulate form is preferable as stated earlier.

It is preferable that the particulate polyvinyl acetal porous material of the present invention is formed into a compressed product as noted above. Especially preferable is a particulate sponge formed of a polyvinyl acetal porous material, characterized in that it has a water content of 10% or less and is rapidly swollen to a 2- to 10-fold volume and has a size in a hydrous condition of from 1 mm to 20 mm when charged into water.

Such a particulate sponge can be produced by a compression step and a drying step. When this particulate sponge is charged into a treatment tank as a microbial support, it rapidly absorbs water and is returned to the original shape and size. At the same time, this particulate sponge is swollen, and compatible with a liquid to be treated. Soon, it can float, sink and be fluidized. Meanwhile, in a particulate sponge formed of a non-compressed polyvinyl acetal porous material, air taken in is hardly separated therefrom, and floats on the surface of water, so that it takes time to fluidize the same. Further, the volume of the porous material is decreased by the compression, and a transportation cost can also be reduced much as stated above.

It is advisable that the compression step here is conducted after the drying step. Even if the compression is conducted in a wet condition, the product is soon returned to the original shape. A particulate sponge formed of a polyvinyl acetal porous material which is produced under appropriate conditions is compressed while it is dried to the water content of 10% or less. As a result, the product can be stored in the compressed state for a long period of time, and it is rapidly swollen when charged into water, and returned to the original shape and size.

A higher compression ratio is better, and the range of from 1/2 to 1/10 is preferable. The particulate sponge compressed to a compression ratio of from 1/2 to 1/10 is, when charged into water, quickly swollen to between 2 and 10 times, and returned to the original size and shape. The size of the particulate sponge in the hydrous condition is preferably between 1 and 20 mm for the above-mentioned reason when it is used as a microbial support.

When it is extremely required that the support is used immediately after charged into a treatment tank, a polyvinyl acetal porous material in a hydrous condition is preferable.

Such a particulate sponge is preferably used as a microbial support, and also as an enzyme immobilization support, a solution-retaining material or a plant support material in a water culture of crops, a culture medium of animal and plant cells, an artificial peatmoss, a soil amelioration material and the like. Further, it can be used as an underwater flowing-type washing member which is a special use example. The underwater flowing-type washing material means as follows. In order to wash vegetables having a delicate surface or vegetables having quite a rough surface, an underwater flowing-type washing material is charged into water along with vegetables, and bubbles are generated from the bottom of the water tank to conduct overall stirring with bubbling. In this manner, the bubbles are brought into contact with the vegetables to wash their surfaces.

A method of producing the polyvinyl acetal-type particulate porous microbial support is not particularly limited. The polyvinyl acetal-type particulate porous microbial support can be obtained by adding a pore forming agent to a polyvinyl alcohol aqueous solution, conducting the reaction with the addition of formaldehyde in the presence of an acid catalyst, then removing the pore forming agent to form a polyvinyl acetal porous material, slicing this porous material to form a sheet, and further cutting the same in the particulate form.

In order to further form this microbial support into an entrap-immobilization microbial support, the polyvinyl acetal porous material is charged into a liquid having dispersed therein an activated sludge, and microbes are adhered to the surface of, and/or in the pores of, the polyvinyl acetal porous material, making it possible to immobilize the same. Further, for example, when a microbe immobilization agent is used to immobilize microbes, the microbe immobilization agent can be dispersed into an activated sludge, and the porous material be charged thereinto to immobilize microbes. In this case, when the porous material is compressed, the sludge is preferably permeated quickly into the particulate material.

Especially when sodium alginate is used as the immobilization agent, the microbial support of the particulate polyvinyl acetal porous material is dipped into a mixed solution of sodium alginate containing microbes. Further, a polyvalent metal salt aqueous solution such as a calcium chloride aqueous solution is added to the microbial support obtained by the dipping into this sodium alginate mixed solution to insolubilize the above-mentioned sodium alginate on the surface of, and/or in the pores of, the porous material, making it possible to obtain a particulate porous microbial support containing the microbes entrapped and immobilized therein. It is preferable that the microbe immobilization agent is caused to flow into the pores of the microbial support under reduced pressure.

The microbial support of the polyvinyl acetal porous material in the present invention can be applied to any known microbial treatment unit. It can be utilized by being filled in a microbial treatment tank whether it is a monolayer type or a multilayer type, and it acts effectively in either anaerobic treatment or aerobic treatment. Further, the microbial support of the present invention is preferably used not only as a support of a fluidized-bed type but also as a support of a so-called fixed-bed type which is immobilized within a reaction tank.

The polyvinyl acetal porous material used in the present invention is obtained by reacting polyvinyl alcohol with an aldehyde. The polyvinyl alcohol resin as a starting material of the polyvinyl acetal porous material is not particularly limited. It is preferable that an average degree of polymerization is between 500 and 3,800, and that the resin is a completely saponified resin or this resin containing a partially saponified material and a low-molecular material. When the average degree of polymerization is less than 500, a product having a high porosity or a product having a porosity of 80% or more which is preferable for the above-mentioned reasons can hardly be obtained. When the average degree of polymerization exceeds 3,800, the viscosity becomes too high when the resin is dissolved in water. Accordingly, it is hard to handle in the production steps such as kneading, defoaming, transportation and the like.

It is also possible to use polyvinyl alcohol resin starting materials having different degrees of polymerization through blending. Further, for example, a resin having a degree of polymerization of 1,500 and a resin having a degree of polymerization of 300, deviating from the above-mentioned range, can be mixed and used too. Especially, a polyvinyl alcohol resin having a low degree of polymerization can also be used to provide a resilience, a flexibility, a feeling such as a touch, a pore diameter controlled, a water absorption improved and the like.

Examples of the aldehyde include aliphatic and aromatic aldehydes such as formaldehyde, benzaldehyde, acetaldehyde, butyl aldehyde, acrylaldehyde and glyoxal. An acetal which is easily converted into an aldehyde with a coexistent acid may be used. Considering a reactivity with polyvinyl alcohol, a strength of a reaction product, a water solubility, a cost, a handleability and an ease of treatment after a reaction, formalin is especially preferable.

Further, in the reaction between an aldehyde and polyvinyl alcohol, it is preferable that an acid catalyst is present. Of course, it is also possible that the acid catalyst is absent. However, the presence of the acid catalyst is effective for accelerating the reaction. The acid is not particularly limited. For example, inorganic and organic acids such as sulfuric acid, hydrochloric acid and maleic acid can selectively be used.

As the microbe entrap-immobilization agent, acrylamide, agar, polyvinyl alcohol or the like is mentioned. A product obtained by using sodium alginate and dipping this into an aqueous solution of a polyvalent metal salt such as calcium chloride or the like is preferable in consideration of a wear resistance and the like. Especially when sodium alginate is used as a main component, the product is easily filled and immobilized well in the polyvinyl acetal porous material, especially the polyvinyl formal porous material, and it is thus compatible with this porous material. Further, the product is preferable in that it is less toxic to immobilization microbes, can be immobilized in a water-soluble material at room temperature and is excellent in a permeability of a waste water. Accordingly, any microbe entrap-immobilization agents other than sodium alginate can be employed if they have the above-mentioned properties.

The microbes used in the present invention are not particularly limited. Bacteria which are used in a sewage treatment unit or a bioreactor can be used without problem. For example, photosynthetic bacteria that decompose organic materials in a liquid to be treated within a treatment tank, denitrifying bacteria and nitrifying bacteria can be mentioned.

The porosity and the degree of acetalization in the present invention including Examples to be described later are measured by the following methods.

(Measurement of a porosity)

A sample was dried (60°C, 3 hours). Then, an apparent volume (Va) was measured using calipers, and a true volume (V) using a dry automatic density meter, Acupick 1330 (trade name) manufactured by Shimadzu Corp., respectively. A porosity $\varepsilon$ (%) was calculated from the resulting value using the following equation.

$$\varepsilon = (1 - V/V_a) \times 100$$

$V_a$ :    apparent volume of a sample

V :    true volume

(Measurement of a degree of acetalization)

Proton NMR measurement in deuterium chloroform and a trifluoroacetic acid aqueous solution was conducted, and a degree F (%) of acetalization was calculated using the following equation.

$$F = (a/c) \times 10$$

c :    total of peak intensities of methylene protons (for example, 4.153, 4.442 ppm)

a:    total of peak intensities of methylene protons (for example, 4.667, 5.150, 5.313, 5.326 ppm) adjacent to an ether group

The present invention is illustrated by referring to the following Examples. However, the present invention is not limited thereto.

(Example 1)

Two-hundred grams of a completely saponified polyvinyl alcohol resin having an average degree of polymerization of 1,500 and 730 g of a partially saponified polyvinyl alcohol resin were charged into water to adjust the total amount to 7 liters. Subsequently, this solution was heated to approximately 100° C, and thoroughly stirred to completely dissolve polyvinyl alcohol. Water was then added thereto to adjust the total amount to 8.5 liters. To this solution was added a starch dispersion obtained by dispersing 500 g of corn starch as a pore forming agent into water to adjust the total amount to 1.5 liters, and these were mixed while being stirred. Thereafter, 900 ml of 50% sulfuric acid and 2 liters of a 37% formaldehyde aqueous solution were continuously added thereto, and the mixture was quickly stirred uniformly to obtain a reaction solution.

The resulting reaction solution was poured into a mold, and reacted at 60°C for 18 hours. Then, the reaction product was withdrawn therefrom, and washed with water to remove the pore forming agent. Thus, a polyvinyl acetal porous material was obtained.

The resulting polyvinyl acetal porous material was sliced to a thickness of 3 mm to form a sheet, and this sheet is further cut to a width of 3 mm to give a ribbon-shaped polyvinyl acetal porous material. This was further successively cut to a length of 3 mm to obtain a cubic polyvinyl acetal porous material 3 mm square.

The degree of acetalization of this polyvinyl acetal porous material was 58 mol%.

The resulting porous material was a sponge of communicating pores having a network structure which had a good hydrophilic nature and was rich in a flexibility and a resilience in wetting. From the porous materials, ten materials were selected as samples, and properties thereof were measured. Consequently, the porosity was uniformly distributed from 80 to 95%, and the average porosity became 90%. Further, the pore diameter was distributed from 40 to 100 μm, and it was on average 60 μm. The pore diameter in this range was appropriate for maintaining microbes within pores, and it was preferable when the material is used as a support.

The apparent specific gravity in the hydrous condition of this cubic polyvinyl acetal porous material 3.0 mm square was between approximately 1.017 and 1.019 with respect to 10 samples. The water content after centrifugation was 50.4%, and the 50% compression stress was $20 \times 10^3$ N/m². When 20 particles were charged into water of a beaker shaken, these floated on the surface of water for a while, and all of the particles then sunk moderately under the surface of water in 3 minutes and 28 seconds.

Subsequently, the cubic polyvinyl acetal porous material 3.0 mm square as obtained in Example 1 was dried at 60°C for 1 minute. The water content was 3.0%. When this material was pressed at a pressure of $4.9 \times 10^6$ N/m², it was compressed to a height of from 0.75 to 1.5 mm. Of there, 20 particles were charged into water which was shaken, and rapidly absorbed water and were swollen. All of them sunk under the surface of water in 8 seconds. The particles which sunk were taken out, and measured where they were wetted in water with a water content of 50%. Then, all of the particles became cubic particles 3.0 mm square, and returned to the original shape and size when the particles were charged into water. That is, it was identified that when the particles were charged into water, they were rapidly swollen to a 2- to 4-fold volume.

On the other hand, 20 particles of the dried samples before the compression were charged into water of a beaker which was shaken. Then, all of the particles floated on the surface of water. The surfaces of the particles absorbed water but air therein was not removed therefrom. Accordingly, none of the particles sunk under the surface of water even though these were shaken for 2 hours.

In a simulation test in which fluidization was conducted in a bioreactor, the resulting polyvinyl acetal porous particles were filled in a container having a diameter of 150 mm and a height of 400 mm such that the volume reached 10% based on the amount of water, and they were fluidized in water with aeration. Consequently, the particles were uniformly dispersed and fluidized. This fluidization was continuously conducted for 1 month, and the particles were then taken out and observed. The wear or the damage owing to rubbing was not observed at all, and the particles were identified to be rich in the wear resistance.

Further, as an accelerating test for comparison with a single product composed of the other material with respect to a wear resistance, a water-resistant sand paper (No. 100 count) was adhered to an inner surface of a side wall of the same test container as mentioned above, and each of various supports was separately filled therein such that the volume reached 10% based on the amount of water, and was stirred and fluidized in water. The stirring was carried out by continuously conducting for 1 week mechanical fluidization of the support through rotation of a stirring blade thrown into a water tank at a rate of 300 rpm which fluidization was set to cause rubbing with the inner wall, and the condition of the support was observed over the course of time. Consequently, the cubic polyvinyl acetal porous material 3 mm square in the present invention was not worn out due to rubbing, and it was identified that this material was rich in the wear resistance.

Meanwhile, with respect to a polyurethane sponge and a cellulose sponge 3 mm square and a calcium alginate spherical gel 3 mm in diameter which were selected as the other material, it was identified that the surface was erased and worn out after 24 hours. Further, after 1 week, the wear proceeded, the size of all of these materials was reduced to less than half the original size. Incidentally, as the calcium alginate gel, a product formed by solidifying a 1% sodium alginate aqueous solution through dropwise addition to a 2% calcium chloride aqueous solution was used.

Further, the particles were filled in a polypropylene container having a large number of holes each having a mesh size of 2 mm at a rate of 10%, and dipped in an activated sludge aeration tank along with this container. After 1 year, this container was taken out, and the polyacetal particles in this container were observed. Then, no dimensional change due to wear was observed at all. In addition, aerobic microbes were adhered to the surfaces of the particles at a high density, and no corrosion of the support with these microbes was identified.

(Example 2)

Two-hundred grams of a completely saponified polyvinyl alcohol resin having an average degree of polymerization and 730 g of a partially saponified polyvinyl alcohol resin were charged into water to adjust the total amount to 7 liters. Subsequently, this solution was heated to approximately 100°C, and stirred well to completely dissolve polyvinyl alcohol. Water was then added thereto to adjust the total amount to 8.5 liters. Into this solution was added a starch dispersion obtained by dispersing 500 g of a potato starch as a pore forming agent into water to adjust the total amount to 1.5 liters, and these were stirred and mixed. Then, 900 ml of 50% sulfuric acid and 2 liters of a 37% formaldehyde aqueous solution were continuously added thereto, and the mixture was rapidly stirred uniformly to form a reaction solution.

The resulting reaction solution was poured into a frame, and reacted at 60°C for 18 hours. Then, the reaction product was taken out, and washed with water to remove the pore forming agent. Thus, a polyvinyl acetal porous material was obtained.

The thus-obtained polyvinyl acetal porous material was sliced to a thickness of 3 mm to form a sheet. This sheet was further cut to a width of 3 mm to obtain a ribbon-type polyvinyl acetal porous material. This material was still further cut to a length of 3 mm continuously to form a cubic polyvinyl acetal porous material 3 mm square.

Separately, a product was prepared by mixing an activated sludge concentrated to 50 g/liter through centrifugation with 2% sodium alginate at a volume ratio of 1:1, and polyvinyl acetal porous material particles produced by the above-mentioned procedure were dipped thereinto. In order to increase the dipping amount, the mixed solution was passed therein under reduced pressure.

The microbe-entrapped product obtained by dipping the polyvinyl acetal porous material into the mixed solution of sodium alginate and the microbes was further added to a 5% calcium chloride aqueous solution. The mixture was stirred, and reacted in this state for approximately 3 hours. Consequently, sodium alginate contained in the polyvinyl acetal porous material was insolubilized, and the microbes were immobilized in the entrapped state.

The thus-obtained microbe entrap-immobilization support was one in which the alginic acid-type gel was adhered to the pores of the polyvinyl acetal porous material and to the surface thereof. This support had a good hydrophilic nature like the polyvinyl acetal porous material, and it was rich in a flexibility and a resilience in wetting. Further, since the polyvinyl acetal porous material was itself rich in the hydrophilic nature, it was well compatible with the gel, and the gel was uniformly present in the particles. Still further, since the porosity was high and the three-dimensional network structure was provided, the gel in the particles was maintained at a high rate.

In the simulation test in which fluidization was conducted in the bioreactor, the resulting microbe entrap-immobilization support was filled in a container 150 mm in diameter and 400 mm in height, and fluidization was conducted in water with aeration. Then, the particles were uniformly dispersed and fluidized. After this fluidization was continuously conducted for 1 month, the particles were taken out, and observed. However, neither the wear nor the damage due to rubbing was observed at all, and it was identified that the particles were rich in the wear resistance. In addition, the polyvinyl acetal porous material did not at all undergo degradation by corrosion with microbes thereof, and it was identified that the life of the support could be long maintained.

Industrial Applicability

The microbial support of the present invention is a polyvinyl acetal porous material. Therefore, even when it is formed as a particulate support, microbes can be immobilized in a treatment tank at a high density, and a wear resistance, a weatherability (light resistance), a resistance to microbial degradation, a fluidity and a microbial affinity can be increased, and the life of the support is prolonged. Accordingly, it is possible that the support is applied to a sewage treatment unit or a bioreactor, that a support fillability in a treatment tank can be maintained high by filling the support into the treatment tank, and that a treatment efficiency can rapidly be increased. Further, since a rate of exchange of a support is decreased, continuous operation in a reaction treatment tank can be conducted for a long period of time.

A mixture of a microbe immobilization agent containing microbes was dipped within the pores of a polyvinyl acetal porous material for insolubilization, whereby microbes can be immobilized within at least pores of the polyvinyl acetal porous material. Accordingly, the polyvinyl acetal porous material having the wear resistance becomes a so-called skeleton to protect a gel having microbes immobilized therein at a high density and lacking in a wear resistance, making it possible to provide a microbe entrap-immobilization support having a quite excellent wear resistance. Still further, not only a wear resistance, but also a physical strength is improved, so that a life of a microbe entrap-immobilization support is abruptly increased, a rate of exchange of the support is decreased, and long-term continuous operation can be conducted when the support is applied to a sewage treatment unit or a bioreactor.

## Claims

1. A microbial support characterized in that a polyvinyl acetal porous material is used as the microbial support.

2. The microbial support recited in claim 1, wherein the polyvinyl acetal porous material is polyvinyl formal.

3. A microbial support characterized in that a sponge formed of a polyvinyl acetal porous material is used as the microbial support.

4. The microbial support recited in any one of claims 1 to 3, wherein an apparent specific gravity in a hydrous condition of the polyvinyl acetal porous material is between 1.0 and 1.2.

5. The microbial support recited in any one of claims 1 to 4, which is a particulate material having a size in a hydrous condition of from 1 mm to 20 mm.

6. The microbial support recited in any one of claims 1 to 5, wherein the polyvinyl acetal porous material has communicating pores with a porosity of from 50 to 98%.

7. The microbial support recited in any one of claims 1 to 6, wherein the degree of acetalization of the polyvinyl acetal porous material is between 30 and 85 mol%.

8. The microbial support recited in any one of claims 1 to 7, wherein the polyvinyl acetal porous material has a large number of pores with a pore diameter of from 20 to 300 μm.

9. The microbial support, wherein microbes are immobilized on the surface of, and/or in the pores of, the polyvinyl acetal porous material recited in any one of claims 1 to 8.

10. The microbial support, wherein microbes are entrapped and immobilized on the surface of, and/or in the pores of, the polyvinyl acetal porous material recited in any one of claims 1 to 8 with a microbe immobilization agent.

11. The microbial support recited in claim 10, wherein the microbe immobilization agent is composed mainly of sodium alginate.

12. A bioreactor in which the microbial support recited in any one of claims 1 to 11 is used.

13. A fluidized-bed type sewage treatment unit characterized in that the treatment is conducted such that the particulate microbial support recited in any one of claims 1 to 11 floats, sinks and is convected in a treatment tank.

14. A particulate sponge for a microbial support characterized in that the particulate sponge has a water content of 10% or less, and is rapidly swollen to a 2- to 10-fold volume and has a size in a hydrous condition of from 1 mm to 20 mm when charged into water.

15. A process for producing a microbial support, which comprises charging a polyvinyl acetal porous material into a liquid having an activated sludge dispersed therein, and adhering microbes on the surface of, and/or in the pores of, the polyvinyl acetal porous material to immobilize the microbes.

16. A process for producing a microbial support, which comprises dipping a polyvinyl acetal porous material into a mixed solution of a microbe immobilization agent containing microbes, insolubilizing the microbe immobilization agent on the surface of, and/or in the pores of, the porous material to entrap and immobilize said microbes.

**17.** A process for producing a microbial support, which comprises dipping a microbial support of a particulate polyvinyl acetal porous material into a mixed solution of sodium alginate containing microbes, further adding this microbial support obtained by the dipping into the sodium alginate mixed solution to a polyvalent metal salt aqueous solution, and insolubilizing said sodium alginate on the surface of, and/or in the pores of, said porous material to obtain a particulate porous microbial support having said microbes entrapped and immobilized therein.

**18.** A particulate sponge which is formed of a polyvinyl acetal porous material.

**19.** The particulate sponge formed of the polyvinyl acetal porous material as recited in claim 18, wherein the particulate sponge has a water content of 10% or less, and is rapidly swollen to a 2- to 10-fold volume and has a size in a hydrous condition of from 1 mm to 20 mm when charged into water.

**20.** A treating method characterized in that the treatment is conducted by bringing the particulate sponge formed of the polyvinyl acetal porous material as recited in claim 18 into contact with a product to be treated.

**21.** A treatment unit characterized in that the treatment is conducted by bringing the particulate sponge formed of the polyvinyl acetal porous material as recited in claim 18 into contact with a product to be treated.

**EP 0 897 000 A1**

## INTERNATIONAL SEARCH REPORT

| International application No. |
|---|
| PCT/JP97/01420 |

### A. CLASSIFICATION OF SUBJECT MATTER
Int. Cl$^6$ C12N11/08, C02F3/00, C12M1/40

According to International Patent Classification (IPC) or to both national classification and IPC

### B. FIELDS SEARCHED

Minimum documentation searched (classification system followed by classification symbols)

Int. Cl$^6$ C12N11/00-11/18, C02F3/00-3/34, C12M1/00-3/10

Documentation searched other than minimum documentation to the extent that such documents are included in the fields searched

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | JP, 59-220186, A (Shoichi Shimizu), December 11, 1984 (11. 12. 84), Claim; example 1 (Family: none) | 1-4, 12-14, 18-21 |
| A | | 16, 17 |
| Y | JP, 63-202382, A (K.K. Taguma Sogo Kenkyusho), August 22, 1988 (22. 08. 88)(Family: none) | 1-9, 12-14, 18-21 |
| A | | 16, 17 |
| Y | JP, 5-130867, A (Kuraray Co., Ltd.), May 28, 1993 (28. 05. 93), Example 1 (Family: none) | 10, 11, 15 |
| A | | 16, 17 |
| | JP, 7-41516, A (Kuraray Co., Ltd.), February 10, 1995 (10. 02. 95), | |

[X] Further documents are listed in the continuation of Box C.　　　[ ] See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| July 23, 1997 (23. 07. 97) | August 5, 1997 (05. 08. 97) |

| Name and mailing address of the ISA/ | Authorized officer |
|---|---|
| Japanese Patent Office | |
| Facsimile No. | Telephone No. |

Form PCT/ISA/210 (second sheet) (July 1992)

12

**INTERNATIONAL SEARCH REPORT**

International application No.

PCT/JP97/01420

| C (Continuation). DOCUMENTS CONSIDERED TO BE RELEVANT |
|---|

| Category* | Citation of document, with indication, where appropriate, of the relevant passages | Relevant to claim No. |
|---|---|---|
| Y<br>A | Example 4 (Family: none) | 10, 11, 15<br>16, 17 |